# EUROPÄISCHE PATENTSCHRIFT

(11) **EP 1 078 650 B1**
(45) Veröffentlichungstag und Bekanntmachung des Hinweises auf die Patenterteilung: **21.06.2006**
(21) Anmeldenummer: 00250265.6
(22) Anmeldetag: 08.08.2000
(51) Int. Cl.: A61N 1/365

(54) **Ratenadaptiver Herzschrittmacher**
Rate adaptive pacemaker
Stimulateur cardiaque à fréquence asservie

(30) Priorität: 20.08.1999 DE 19940952
(43) Veröffentlichungstag der Anmeldung: 28.02.2001
(73) Patentinhaber: BIOTRONIK GmbH & Co. KG, 12359 Berlin (DE)
(72) Erfinder: Hutten, Helmut, 8010 Graz (AT)
(74) Vertreter: Lindner-Vogt, Karin L.

(56) Entgegenhaltungen:
- EP-A- 0 089 014
- EP-A- 0 449 401

## Beschreibung

Die Erfindung betrifft einen ratenadaptiven Herzschrittmacher mit Fühlermitteln zur Erfassung mindestens einer zur Kreislauffunktion in Beziehung stehenden Meßgröße im Körper des Patienten, Stimulationsmitteln zur Erzeugung und Abgabe von Stimulationsimpulsen an das Herz des Patienten und Ratensteuermitteln, die eingangsseitig mit den Fühlermitteln und ausgangsseitig mit den Stimulationsmitteln verbunden sind, zur Steuerung der Rate der Stimulationsimpulse in Abhängigkeit von der zur Kreislauffunktion in Beziehung stehenden Meßgröße.

Frequenz- oder ratenadaptive Herzschrittmacher sind in großer Mannigfaltigkeit bekannt und seit langem im klinischen Einsatz. Da sie auf Änderungen der physiologischen Bedürfnisse des Patienten mit Änderungen der Stimulationsfrequenz reagieren können, kann wechselnden Belastungszuständen des Patienten wirksam Rechnung getragen werden.

Die Konstruktion eines ratenadaptiven Schrittmachers kann auf sehr verschiedenen Steuer- und regelungstechnischen Ansätzen beruhen. Sie ist grundsätzlich um so erfolgversprechender, je weitgehender sie sich die physiologischen Zusammenhänge zwischen Belastung und Pulsfrequenz beim Patienten mit gesundem Herzen zunutze macht.

Besonders vorteilhaft sind unter diesem Gesichtspunkt sinusknoten-gesteuerte Geräte, welche direkt die elektrische Sinusknoten- bzw. Vorhofaktivität abfühlen. Diese vorhof-synchronen Schrittmacher werden bei Krankheitsbildern mit atrioventrikulärer Leitungsstörung bei intakter Sinusknotenfunktion regelmäßig eingesetzt. Bei ihnen wird der Absolutwert der Stimulationsrate jederzeit unmittelbar durch das körpereigene Reizbildungssystem unter Führung durch das Zentralnervensytem (ZNS) vorgegeben.

Anders liegen die Dinge bei frequenzadaptiven Schrittmachern, die bei der Therapie von Krankheitsbildern mit Störungen der Sinusknotenfunktion einsetzbar sind: Derartige Schrittmacher nutzen in verschiedener Weise Sensor-Informationen über die physiologischen Bedürfnisse des Patienten und/oder dessen physische Aktivität. Diese Meßwerte und die daraus ermittelten Änderungen der Stimulationsfrequenz sind jedoch grundsätzlich relativer Natur - vgl. etwa US 3 593 718, die eine der ersten Beschreibungen eines (atmungsgesteuerten) ratenadaptiven Schrittmachers gibt. Das heißt, eine Information über den physiologisch korrekten Absolutwert der Stimulationsfrequenz liegt bei derartigen Schrittmachern nicht vor. Dennoch kann deren Funktion auf der Basis dieser relativen Stimulationsfrequenzgröße in vielen Anwendungsfällen zufriedenstellen, da Fehleinstellungen der absoluten Stimulationsfrequenz durch die körpereigene Regelung des Herzschlagvolumens ausgeglichen werden. Der Ausgleich von Raten-Fehleinstellungen durch das Herzschlagvolumen stellt jedoch eine dauerhafte zusätzliche Belastung des Patienten dar und hat zudem bei Patienten mit verminderter Volumenadaptionsfähigkeit natürlich gewisse Grenzen.

Beispielsweise in US 4 884 576 wird eine patientenspezifische Kalibrierung der zur Bestimmung der Stimulationsrate dienenden Kennlinie zwischen einer unteren und einer oberen Grenzrate vorgeschlagen, wobei hier speziell der Langzeit-Mittelwert des Respirationssignals zur Festlegung der Gestalt der Kennlinie und optional zusätzliche Signale zu einer Verschiebung innerhalb des fest vorgegebenen Stimulationsfrequenzbandes dienen sollen.

Aus EP 0 449 401 ist ein ratenadaptiver Schrittmacher bekannt, bei dem die jeweilige aktuelle Stimulationsrate in Abhängigkeit von der jeweils aktuellen Änderung der Respirationsrate und der Respirationstiefe gebildet wird.

Aus EP 0 089 014 ist ebenfalls ein ratenadaptiver Schrittmacher bekannt bei dem die Stimulationsrate in Abhängigkeit der Respirationsrate gebildet wird.

Die der Erfindung zugrundeliegende Aufgabe besteht in der Bereitstellung eines gattungsgemäßen Herzschrittmachers, bei dem Fehleinstellungen der absoluten Stimulationsrate über den gesamten relevanten Einstellbereich vermieden werden.
Die Aufgabe wird durch einen Herzschrittmacher mit den im Anspruch 1 angegebenen Merkmalen gelöst.

Die Erfindung schließt den Gedanken ein, den Schrittmacher mit Mitteln zur Gewinnung einer Sensor-Information über den physiologisch korrekten Absolutwert der Stimulationsfrequenz auszustatten. Da für die hier in Rede stehenden Schrittmachertypen eine natürliche Herzrateninformation nicht verfügbar ist, gehört zur Erfindung weiterhin der Gedanke, an deren Stelle eine Sensor-Information über eine sonstige zentralnervös geregelte Größe zu setzen, die verfügbar ist. Einen vorteilhaften Ansatzpunkt hierfür bietet die zentralnervöse Kopplung von Respirations- und Kreislauftätigkeit, bei der als kreislaufrelevante Größen vor allem Herzfrequenz, Herzschlagvolumen und Vasomotorik (hier vor allem der Gefäßwiderstand) von Bedeutung sind. Da die Schrittmacherrate möglichst genau die physiologische Herzrate nachbilden soll, schließt die Erfindung insbesondere den Gedanken ein, einen physiologisch korrekten Absolutwert der Stimulationsrate mittels geeigneter Verarbeitung eines Respirationssignals zu gewinnen.

Beim gesunden menschlichen Körper stellt sich vorrangig in Ruhephasen, speziell während des Nachtschlafes, eine enge Koppelung zwischen Atemtätigkeit und Grund-Herzrate innerhalb bestimmter Frequenzverhältnisse ein. Das sich einstellende Frequenzverhältnis kann davon abhängig sein, ob der Patient sportlich aktiv ist.

Tendentiell stellt sich beispielsweise für Ausdauersportler ein Herz-/Atemfrequenz-Verhältnis von etwa 3 ein, für sportlich weniger aktive Menschen eines von etwa 4.

Vorteilhafterweise wird zur Erfassung der Respirationssignale aufgrund der einfachen technischen Realisierbarkeit ein hinsichtlich der Signalauswertung modifizierter Impedanzsensor (Impedanzpneumograph) vorgesehen, der als solcher seit langem bekannt und für Schrittmacher etwa in EP 0 151 689 B1 oder EP 0 249 818 A1 beschrieben ist. Die Messung der Respirationsrate kann entweder alternativ oder gemeinsam durch Messung der intrathorakalen und/oder der intrakardialen Impedanz erfolgen. Falls sowohl die intrathorakale als auch die intrakardiale Impedanz gemessen werden, können diese Messungen zur gegenseitigen Signalbereinigung herangezogen werden.

In Anbetracht der oben erwähnten Zusammenhänge sind zunächst durch den Schrittmacher (nächtliche) Ruhephasen zu detektieren. Diese können aufgrund der Informationen anderer Sensoren erkannt werden, etwa durch Erkennung von Rapid-Eye-Movement-Phasen herangezogen. In EP 0 502 918 B1 werden Aktivitätssensordaten zur Detektion von Ruhe- bzw. Schlafphasen herangezogen.

Besonders sinnvoll ist es jedoch, den zur Gewinnung der relevanten Meßdaten vorgesehenen Respirationsratensensor auch zur Detektion nächtlicher Ruhephasen zu verwenden. Schlafphasen können hiermit in der Regel anhand einer bestimmten Höhe und vor allem einer bestimmten Stabilität der Respirationsrate detektiert werden.

Der Ratenadaptionsalgorithmus des Herzschrittmachers kann von Änderungen der Basis-Stimulationsrate unabhängig beibehalten werden. Die aufgrund des Algorithmus ermittelte Rate - gewissermaßen der dynamische Anteil - wird dann entsprechend einem aus dem Respirationssignal ermittelten Offset - dem statischen Anteil - zu höheren oder niedrigeren Raten hin verschoben. Dann ist zu erwarten, daß sich in ähnlichem Maße wie die Basis-Stimulationsrate auch die durchschnittliche Stimulationsfrequenz verändert.

Es besteht andererseits die Möglichkeit, den Ratenadaptions-Algorithmus so zu verändern, daß bei Veränderungen der Basis-Stimulationsrate weiterhin die ursprüngliche Maximalrate erreicht wird. Dazu muß umgekehrt proportional zu Änderungen der Basis-Stimulationsrate die Steigung der Ratensteuerkennlinie angehoben oder verringert werden.

Besonders vorteilhaft kann die Erfindung bei Herzschrittmachern angewendet werden, die zur dynamischen Ratenadaption komplexe Kurvenformen wie den zeitlichen Verlauf der intrakardialen Impedanz, die ventrikulär evozierte Reizantwort (VER = ventricular evoked response) oder monophasische Aktionspotentiale (MAP) auswerten. Bei der Auswertung solcher Kurven zur Ableitung von Ratensteuersignalen besteht häufig die Schwierigkeit, daß ein Referenz-Kurvenverlauf für den ruhenden Patienten gefunden werden muß, der mit der Grund-Stimulationsrate des Herzschrittmachers zu korrelieren ist. Die Erfindung ermöglicht bei solchen Herzschrittmachern das gleichzeitige Erfassen eines Referenzkurvenverlaufes und der zugehörigen Grund-Stimulationsrate.

Eine weitere Fortbildung der Erfindung im Hinblick auf solche Herzschrittmacher, die komplexe Kurvenformen von Herzaktivitätssignalen auswerten, besteht darin, daß auch ein mittlerer stabiler Belastungszustand anhand der Respirationstätigkeit (d.h. deren Rate = Atemfrequenz und Hub = Atemvolumen) detektiert wird, um eine Referenzkurve für mittlere Belastungszustände aufzeichnen zu können. Da mit zunehmender Belastung nämlich die Kopplung zwischen Respirations- und Herzrate verschwindet, ist die absolute Bestimmung einer Herzrate bei mittleren Belastungen allein aufgrund der Respirationsrate nicht mehr möglich, und zur Detektion des Belastungszustandes müssen Respirationsrate und -hub erfaßt werden. Hiermit ist dann allerdings auch eine weitere Stützstelle zur Bestimmung der Parameter des Ratenadaptionsalgorithmus zu gewinnen, und es wird eine vollständige Autokalibration des Ratenadaptionsalgorithmus allein mit Hilfe eines Respirationssensors ermöglicht.

Vorteilhafte Weiterbildungen der Erfindung sind im übrigen in den Unteransprüchen gekennzeichnet bzw. werden nachstehend im Zuge der Beschreibung bevorzugter Ausführungen der Erfindung anhand der Figuren näher dargestellt. Es zeigen:
- Figur 1: ein Funktions-Blockschaltbild eines Herzschrittmachers gemäßeinem bevorzugten Ausführungsbeispiel der Erfindung und
- Figur 2: ein Funktions-Blockschaltbild einer Ausführungsform der Kennlinien-Einstellvorrichtung des Schrittmachers nach Fig. 1.

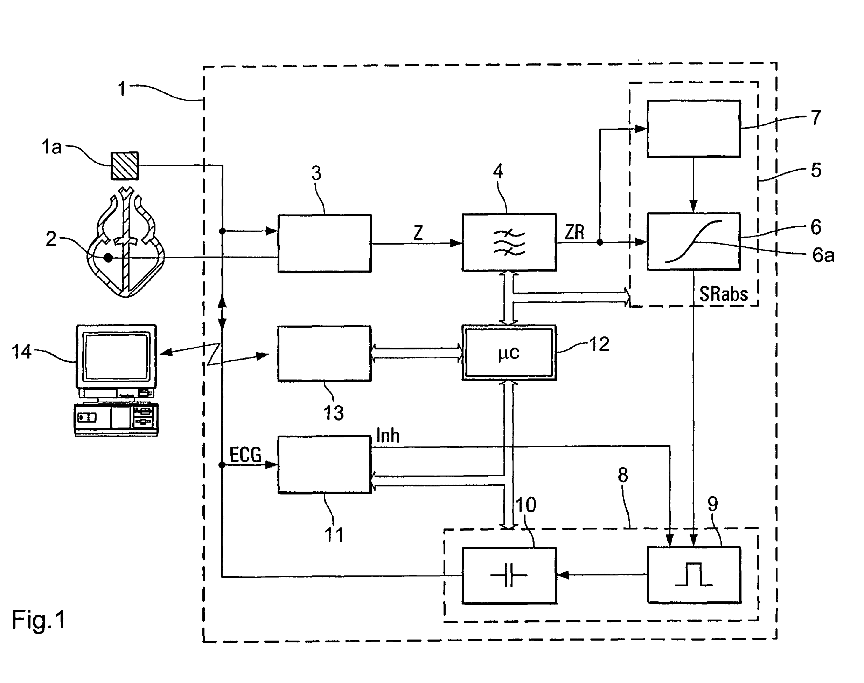

Fig. 1 zeigt ein schematisches Funktions-Blockschaltbild, in dem nur die für die Erläuterung der Erfindung wichtigen Komponenten dargestellt sind, einer Schrittmacheranordnung mit einem ratenadaptiven Herzschrittmacher 1.

Der Schrittmacher 1 umfaßt eine - als solche bekannte - Impedanzmeßeinrichtung 3, die eingangsseitig mit einer Gehäuseelektrode 1a sowie einer unipolaren Elektrode 2 im rechten Ventrikel eines Herzens H verbunden ist und die Impedanz Z der Meßstrecke erfaßt. Die Messung erfolgt, indem die Elektrode 2 mit einer (nicht-stimulierenden) Meßspannung mit einer Takfrequenz von einigen zehn bis ca. 100 Hz beaufschlagt wird und in vorbestimmten Zeitabständen innerhalb eines fest vorprogrammierten Zeitbereiches der Strom zwischen den Elektroden 1a und 2 erfaßt wird. Die Impedanz Z ergibt sich als Quotient aus Meßspannung und Strom. Der Meßeinrichtung 3 nachgeschaltet ist eine Impedanzsignalbearbeitungsstufe 4 mit Bandfiltereigenschaften zur Ausfilterung höherfrequenter, nicht respirationsbedingter Impedanzänderungen und zur Kompensation von Nulliniendrift, die ein die Respirationsrate widerspiegelndes Ausgangssignal ZR liefert.

Das Ausgangssignal ZR der Signalverarbeitungsstufe 4 wird einer Ratenbestimmungseinrichtung 5 zugeführt, die ein Ratensteuersignal SRabs ausgibt, das den Absolutwert der adaptiven Stimulationsrate festlegt.

Die Ratenbestimmungseinrichtung 5 weist ein Kennlinienglied 6 auf, das jedem Wert des Respirationsratensignals ZR mittels einer Kennlinie 6a einen Wert der adaptiven Stimulationsrate SRabs zuordnet. Die Kennlinie wird während des Betriebs des Herzschrittmachers periodisch durch eine Kennlinien-Einstellvorrichtung 7 optimiert, wobei (optional) auch jeweils die zulässige Variationsbreite der Stimulationsrate SRabs zwischen einer Grundrate SRmin als unterem Grenzwert und einer maximalen Stimulationsrate SRmax als oberem Grenzwert neu bestimmt wird. Der Ratenbestimmungseinrichtung 5 ist eine in üblicher Weise ausgebildete Impulserzeuger- und Ausgangsstufe 8 nachgeschaltet, die eine Impulserzeugerstufe 9 und eine mit deren Ausgang verbundene Ausgangsstufe 10 aufweist, welche zur Ausgabe eines Stimulationsimpulses mit der (nach obigem gleichzeitig als Meß- und Reizelektrode dienende) Elektrode 2 verbunden ist. Die Impulserzeuger- und Ausgangsstufe 8 ist über einen weiteren Steuereingang mit dem Ausgang einer Herzsignalerfassungsstufe 11 verbunden, die über die Elektrode 2 ein intrakardiales Elektrokardiogramm (ECG) am Herzen abnimmt und daraus ggfs. ein Inhibierungssignal Inh ableitet, durch das bei ausreichender Eigenfrequenz die Ausgabe eines Stimulationimpulses inhibiert werden kann.

Zur Steuerung der allgemeinen Schrittmacherfunktionen sowie zur Durchführung der Impedanzmessung und -verarbeitung ist eine Ablaufsteuerung (Controller) 12 vorgesehen, die in Datenbusverbindung mit den übrigen Schrittmacherkomponenten sowie einer Telemetrieeinheit 13 steht, über die in an sich bekannter Weise ein Datenaustausch mit einem externen Auswertungs- und Programmiergerät 14 erfolgen kann.

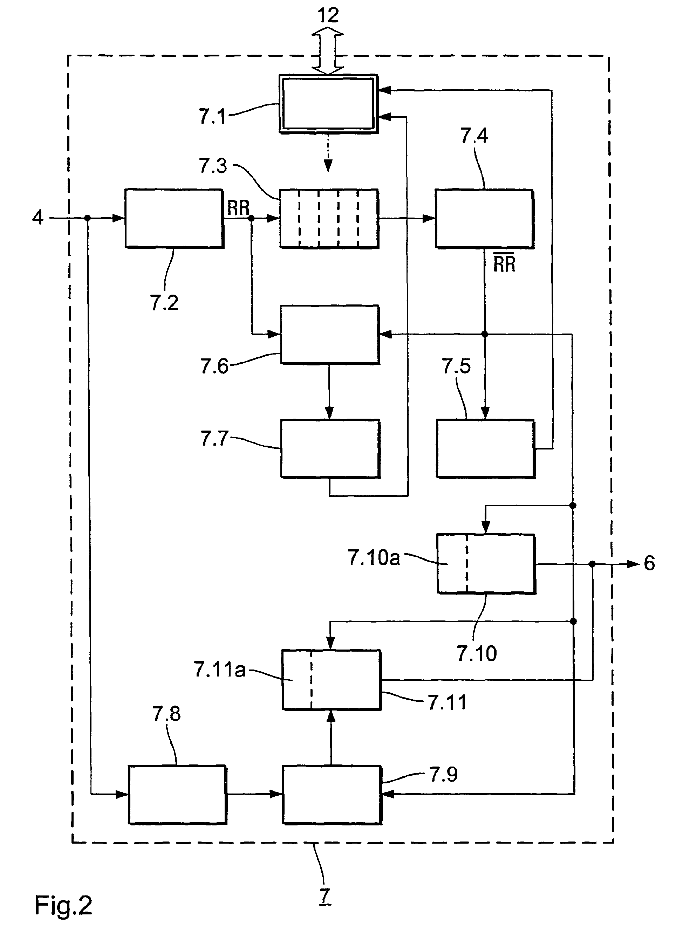

Fig. 2 zeigt in einem Funktions-Blockschaltbild die für die Ausführung der Erfindung wesentlichen Komponenten einer Kennlinien-Einstellvorrichtung 7 des Schrittmachers 1. Hierbei ist zu beachten, daß die Kalibrierung eines sensorgestützten ratenadaptiven Schrittmachers als solche dem Fachmann vertraut ist und die entsprechenden Grundfunktionen hier nicht näher beschrieben werden. Der Fachmann geht im übrigen davon aus, daß die beschriebenen Funktionen bei einem modernen Schrittmacher mindestens teilweise softwaremäßig realisiert werden.

Die Kennlinien-Einstellvorrichtung 7 umfaßt eine interne Ablaufsteuerung 7.1, die einerseits über eine Datenbusverbindung an den Controller 12 und speziell an dessen Zeitbasis und andererseits (über nicht einzeln dargestellte Steuerverbindungen) an die unten erwähnten Komponenten angebunden ist und die Ausführung der nachfolgend beschriebenen Schritte in auf den allgemeinen Schrittmacherbetrieb abgestimmter Weise steuert.

Sie umfaßt weiter eine eingangsseitig mit der Impedanzsignalbearbeitungsstufe 4 (Fig. 1) verbundene Respirationsraten-Bestimmungseinheit 7.2 und einen dieser nachgeschalteten Respirationsratenspeicher 7.3 sowie - jeweils eingangsseitig mit dem Speicher 7.3 verbunden - eine Mittelungsstufe 7.4, einen Respirationsratendiskriminator 7.5 und eine, zusätzlich mit der Mittelungsstufe 7.4 verbundene, Arithmetikeinheit 7.6 zur Ausführung verschiedener Berechnungen hinsichtlich der Respirationsrate sowie einen mit der Arithmetikeinheit verbundenen Variabilitäts-Diskriminator 7.7.

Weiter umfaßt die Kennlinien-Einstellvorrichtung 7 eine eingangsseitig ebenfalls mit der Signalbearbeitungsstufe 4 verbundene Respirationshub-Bestimmungseinheit 7.8 und eine dieser nachgeschaltete Respirationshub-Verarbeitungsstufe 7.9, und ferner eine Grundraten-Stelleinheit 7.10 mit zugeordnetem Verhältniswertspeicher 7.10a und eine Steigungs-Stelleinheit 7.11 mit zugeordnetem Kennlinienformspeicher 7.11a, die ausgangsseitig mit dem Kennlinienglied 6 verbunden sind. Die Grundraten-Stelleinheit 7.10 ist eingangsseitig zudem mit dem Ausgang der Mittelungsstufe 7.4 verbunden, und die Steigungs-Stelleinheit 7.11 neben diesem zusätzlich mit dem Ausgang der Respirationshub-Verarbeitungsstufe 7.9.

Die Einstellung der Ratensteuerkennlinie erfolgt mittels dieser Komponenten auf die folgende bevorzugte Weise:

Zunächst erfolgt die Detektion einer nächtlichen Ruhephase anhand der Zugehörigkeit der Respirationsrate zu einem bestimmten Wertebereich und der Erfüllung eines bestimmten Stabilitätskriteriums. Ein solcher Detektionsvorgang kann, falls der Controller 12 einen Echtzeitgeber aufweist, zu bestimmten Uhrzeiten oder auch einfach in vorbestimmten Zeitabständen durch die interne Ablaufsteuerung 7.1 ausgelöst werden. Daraufhin wird für eine durch die Kapazität des Respirationsratenspeichers 7.3 bestimmte Anzahl von Atemzügen fortlaufend durch die Stufen 7.2 bis 7.4 die momentane Atemfrequenz (Respirationsrate) bestimmt und gespeichert sowie gemittelt. Im Respirationsratendiskriminator wird der Mittelwert einer Diskriminierung hinsichtlich der Zugehörigkeit zu einem vorbestimmten Frequenzbereich unterzogen, und zudem wird in der Arithmetikeinheit 7.6 sowie dem Variabilitäts-Diskriminator 7.7 die Variabilität der Frequenz der vorbestimmten Anzahl von Atemzügen bestimmt und auf Unterschreitung eines vorbestimmten Schwellwertes geprüft.

Als Kriterien für das Vorliegen einer Schlafphase dient das Auftreten einer Gruppe von 4 bis 8 Atemzügen mit einer Raten-Variabilität nicht über 10% und einer Rate innerhalb eines Bereichs von 15 bis 25 pro Minute. Die Erfüllung der Kriterien wird innerhalb einer in den Ablaufsteuerung 7.1 programmierten Testperiode geprüft, und der Test wird entweder bei Erfüllung beider Kriterien oder nach Ablauf der Testperiode beendet. Bei positivem Ergebnis werden die nachfolgend beschriebenen Schritte ausgeführt, bei negativem Ausgang wird die gültige Ratensteuerkennlinie bis auf weiteres beibehalten.

Wurde eine Schlafphase detektiert, wird die gemittelte Respirationsrate an die Grundraten-Stelleinheit 7.10 übergeben und dort mit einem im zugehörigen Speicher 7.10a gespeicherten Faktor zwischen 2,5 und 4,5 multipliziert, um so die aktuelle Grundrate zu erhalten. Vorteilhafterweise kann der Speicher 7.10a durch den Arzt programmierbar sein. Dadurch kann ein Wert entsprechend der sportlichen Kondition des Patienten gewählt werden, wobei bei Ausdauersport betreibenden Patienten niedrigere Werte gewählt werden als bei inaktiven. (Grundsätzlich besteht - etwa bei einem zusätzlich aktivitätsgesteuerten Schrittmacher mit Histogrammfunktionen - auch die Möglichkeit, den Faktor adaptiv zu machen, indem er bei Patienten, bei denen eine ausgeprägte körperliche Aktivität über lange Zeiträume hinweg detektiert wird, gegenüber eher inaktiven Patienten automatisch niedriger eingestellt oder verringert wird.)

Der Verlauf der Kennlinie kann bei einer Änderung der Grundrate entweder unverändert gelassen werden (was zu einem Offset auch der Maximalrate führt), oder es erfolgt - ebenfalls initiiert durch die Ablaufsteuerung 7.1 - durch die Steigungs-Stelleinheit 7.11 unter Zugriff auf im Kennlinienformspeicher 7.11a gespeicherte Reserve-Kennlinien sogleich eine Verlaufs-Anpassung dahingehend, daß die bisherige Maximalrate beibehalten wird. Die Adressierung der gespeicherten Kennlinien erfolgt anhand des Ausgabewertes der Grundraten-Stelleinheit 7.10. Unabhängig hiervon kann eine Einstellung bzw. Nachjustierung des Kennlinienverlaufes in einer Phase mittlerer Belastung des Patienten anhand einer gemeinsamen Verarbeitung von Respirationsrate und -hub erfolgen. Das Vorliegen einer solchen Phase wird in Analogie zur oben beschriebenen Detektion einer Ruhephase anhand einer bestimmten Bereichszuordnung sowie der Erfüllung eines Variabilitätskriteriums erfaßt. Zusätzlich kann (was aber aus der Figur nicht ersichtlich wird) auch die Prüfung der Erfüllung vorbestimmter Bereichs- und Variabilitätskriterien für den Respirationshub zweckmäßig sein.

Wurde ein Zustand stabiler mittlerer Belastung detektiert, werden der parallel zur Respirationsrate in der Respirationshub-Bestimmungseinheit 7.8 ermittelte Respirationshub und die zugehörigen Atemfrequenzsignale von der Mittelungsstufe 7.4 oder der Arithmetikeinheit 7.6 in der Respirationshub-Verarbeitungsstufe 7.9 einer gemeinsamen Verarbeitung nach einem vorgegebenen Algorithmus unterzogen. Die Einzelheiten eines solchen Algorithmus gehören aber nicht zur vorliegenden Erfindung und werden daher hier nicht beschrieben.

Die Erfindung beschränkt sich in ihrer Ausführung nicht auf die vorstehend angegebenen bevorzugten Ausführungsbeispiele. Vielmehr ist eine Vielzahl von Varianten möglich, welche von der dargestellten Lösung auch in anders gearteten Ausführungen Gebrauch machen.

So ist die Erfindung im Sinne eines qualifizierten Zwei-Sensoren-Konzeptes insbesondere auch bei Schrittmachern einsetzbar, die einen weiteren Fühler für eine belastungsabhängige Größe mit einer für diesen spezifischen Ratensteuerkennlinie aufweisen. Sie ermöglicht in diesem Zusammenhang die physiologisch korrekte Zuordnung einer Grundrate bzw. eines Kennlinien-Startpunktes zum gegebenen Kennlinienverlauf und damit die Überführung der in der Beschreibungseinleitung erwähnten relativen Ratensteuerung in eine Absolutwert-Steuerung. In der zuletzt angesprochenen Variante mit zusätzlicher Respirationshub-Auswertung in einem mittleren Belastungszustand wird dann zusätzlich eine Verifizierung der Kennliniengestalt möglich, die die physiologische Qualität der Steuerung weiter erhöht. Weiterhin ist die Nutzung der in bestimmten körperlichen Zuständen eng mit der Herzrate korrelierenden Größe - beispielsweise der Respirationsrate und ggfs. auch des Respirationshubes - nicht auf die Steuerung der Stimulationsrate beschränkt, sondern sie kann bei einem Zweikammerschrittmacher in ähnlicher Weise auch zur Steuerung des AV-Intervalls herangezogen werden.

## Patentansprüche

1. Ratenadaptiver Herzschrittmacher (1) mit Fühlermitteln (1a bis 4) zur Erfassung mindestens einer zur Kreislauffunktion in Beziehung stehenden Messgröße (RR) im Körper des Patienten, Stimulationsmitteln (8, 2) zur Erzeugung und Abgabe von Stimulationsimpulsen an das Herz (H) des Patienten und Ratensteuermitteln (5), die eingangsseitig mit den Fühlermitteln und ausgangsseitig mit den Stimulationsmitteln verbunden sind, zur Steuerung der Rate der Stimulationsimpulse (SRabs) in Abhängigkeit von der zur Kreislauffunktion in Beziehung stehenden Messgröße, wobei die Ratensteuermittel eine Einstellvorrichtung (7) aufweisen, die ausgebildet ist, in einem stabilen Zustand niedriger oder mittlerer körperlicher Belastung eine Einstellgröße für die Einstellung eines Absolutwertes einer unteren Grenzrate (SRmin) der Stimulationsimpulse in Abhängigkeit von der Respirationsrate (RR) zu bilden und zur Ratensteuerung derart heranzuziehen, dass die Rate der Stimulationsimpulse (SRabs) von dem Verlauf einer die Abhängigkeit der Rate der Stimulationsimpulse (SRabs) von der Messgröße beschreibenden Ratensteuerkennlinie (6a) und zusätzlich von der während eines stabilen Zustands niedriger oder mittlerer körperlicher Belastung ermittelten Einstellgröße für die Einstellung eines Absolutwertes einer unteren Grenzrate (SRmin) abhängt.

2. Ratenadaptiver Herzschrittmacher nach Anspruch 1, **dadurch gekennzeichnet; dass** die Ratensteuermittel (5) derart ausgebildet sind, dass sie einen dynamischen Anteil der Rate der Stimulationsimpulse in Abhängigkeit einer mit dem aktuellen körperlichen Bedarf einhergehenden Messgrö-βe gegenüber einem statischen Anteil variieren, der über die Einstellvorrichtung (7) von der während einer Ruhephase ermittelten Einstellgröße abhängt.

3. Ratenadaptiver Herzschrittmacher nach Anspruch 1 oder 2, **dadurch gekennzeichnet, dass** die Einstellvorrichtung (7) Zustandserfassungsmittel (7.2 bis 7.7) zur Detektion eines Zustandes niedriger körperlicher Belastung, insbesondere aufgrund eines Vergleiches einer Eigenschaft der Messgröße mit einem vorbestimmten Kriterium, aufweist.

4. Ratenadaptiver Herzschrittmacher nach Anspruch 3, **dadurch gekennzeichnet, dass** die Zustandserfassungsmittel (7.2 bis 7.7) Mittel (7.2 bis 7.5) zur Feststellung der Zugehörigkeit von aktuellen Werten der Messgröße zu einem vorbestimmten Wertebereich aufweisen.

5. Ratenadaptiver Herzschrittmacher nach Anspruch 3 oder 4, **dadurch gekennzeichnet, dass** die Zustandserfassungsmittel (7.2 bis 7.7) Mittel (7.6, 7.7) zur Ermittlung der Variabilität der Messgröße und zum Vergleich mit einem vorbestimmten Grenzwert aufweisen.

6. Ratenadaptiver Herzschrittmacher nach einem der Ansprüche 1 bis 65, **dadurch gekennzeichnet, dass** die Einstellvorrichtung (7) zur Bildung eines Einstellwertes als Offset für den Verlauf einer die Abhängigkeit der Rate der Stimulationsimpulse von der Messgröße beschreibenden Ratensteuerkennlinie (6a) ausgebildet ist.

7. Ratenadaptiver Herzschrittmacher nach einem der vorhergehenden Ansprüche, **dadurch gekennzeichnet, dass** die Einstellvorrichtung (7) Einstellmittel (7.8 bis 7.11) zur Einstellung der unteren Grenzrate (SRmin) und/oder des Verlaufes mindestens eines Abschnitts der Ratensteuerkennlinie (6a) gemäß einer vorbestimmten Beziehung aufweist, die bei Detektion eines stabilen Zustandes niedriger Belastung aktiviert werden.

8. Ratenadaptiver Herzschrittmacher nach einem der vorhergehenden Ansprüche, **dadurch gekennzeichnet, dass** die Messgröße die Respirationsrate (RR) ist und die Fühlermittel (1a bis 4) zu deren Bestimmung aufgrund von Messungen der intrathorakalen Impedanz (Z) ausgebildet sind.

9. Ratenadaptiver Herzschrittmacher nach einem der vorhergehenden Ansprüche, **dadurch gekennzeichnet, dass** die Messgröße die Respirationsrate (RR) ist und die Fühlermittel (1a bis 4) zu deren Bestimmung aufgrund von Messungen der intrakardialen Impedanz (Z) ausgebildet sind.

10. Ratenadaptiver Herzschrittmacher nach Anspruch 8 oder 9, **dadurch gekennzeichnet, dass** die Einstellvorrichtung derart ausgebildet ist, dass die untere Granzrate (SRmin) und/oder die Rate der Stimulationsimpulse (SRabs) in einem einstellbaren Verhältnis zur Respirationsrate (RR) steht.

11. Ratenadaptiver Herzschrittmacher nach Anspruch 10, **dadurch gekennzeichnet, dass** die Einstellvorrichtung derart ausgebildet ist, dass die untere Granzrate (SRmin) und/oder die Rate der Stimulationsimpulse (SRabs) in einem einstellbaren Verhältnis zu einem längerfristigen Mittelwert der Respirationsrate (RR) steht.

12. Ratenadaptiver Herzschrittmacher nach einem der vorhergehenden Ansprüche, **dadurch gekennzeichnet, dass** die Einstellvorrichtung (7) zu einer zusätzlichen Kalibration der Ratensteuerkennlinie in einem stabilen Zustand mittlerer Belastung ausgebildet ist.

13. Ratenadaptiver Herzschrittmacher nach Anspruch 9 und 10, **dadurch gekennzeichnet, dass** die Fühlermittel zur Erfassung des Respirationshubes und die Zustandserfassungsmittel (7.2 bis 7.7) und/oder die Einstellmittel (7.8 bis 7.11) zu dessen Auswertung neben der Respirationsrate in dem stabilen Zustand mittlerer Belastung ausgebildet sind.

14. Ratenadaptiver Herzschrittmacher nach einem der vorhergehenden Ansprüche, **dadurch gekennzeichnet**, das weitere Fühlermittel zur Erfassung einer weiteren aktivitäts- oder belastungsabhängigen Messgröße vorgesehen sind und die Einstellvorrichtung zur Einstellung der Ratensteuerkennlinie in Abhängigkeit von der weiteren Messgröße ausgebildet ist.

15. Ratenadaptiver Herzschrittmacher nach Anspruch 1, **dadurch gekennzeichnet, dass** die weiteren Fühlermittel zur Erfassung einer belastungsabhängigen Messgröße in einem Herzaktivitätssignal ausgebildet sind.

## Claims

1. A rate-adaptive pacemaker (1) having sensor means (1a through 4) for detecting at least one measured variable (RR), which is related to the circulatory function, in the body of the patient, stimulation means (8, 2) for generating and delivering stimulation pulses to the heart (H) of the patient, and rate control means (5), which are connected at the input to the sensor means and at the output to the stimulation means, for controlling the rate of the stimulation pulses (SRabs) as a function of the measured variable related to the circulatory function, wherein the rate control means have a setting device (7), which is implemented, in a stable state of low or moderate body load, to calculate a setting variable for setting an absolute value of a lower limiting rate (SRmin) of the stimulation pulses as a function of the respiration rate (RR) and used for the rate control in such a way that the rate of the stimulation pulses (SRabs) is a function of the curve of a rate control characteristic curve (6a), which describes the dependence of the rate of the stimulation pulses (SRabs) on the measured variable, and, in addition, of the setting variable for setting an absolute value of a lower limiting rate (SRmin), which is ascertained during a stable state of low or moderate body load.

2. The rate-adaptive pacemaker according to Claim 1, **characterized in that** the rate control means (5) are implemented in such a way that they vary a dynamic component of the rate of the stimulation pulses as a function of a measured variable connected to the current bodily requirement in relation to a static component, which is a function of the setting variable ascertained during a rest phase via the setting device (7).

3. The rate-adaptive pacemaker according to Claim 1 or 2, **characterized in that** the setting device (7) has state detection means (7.2 through 7.7) for detecting a state of low bodily load, particularly on the basis of a comparison of a property of the measured variable to a predetermined criterion.

4. The rate-adaptive pacemaker according to Claim 3, **characterized in that** the state detection means (7.2 through 7.7) have means (7.2 through 7.5) for determining the association of current values of the measured variable with a predefined value range.

5. The rate-adaptive pacemaker according to Claim 3 or 4, **characterized in that** the state detection means (7.2 through 7.7) have means (7.6, 7.7) for ascertaining the variability of the measured variable and for comparison to a predetermined limiting value.

6. The rate-adaptive pacemaker according to one of Claims 1 through 5, **characterized in that** the setting device (7) is implemented to calculate a setting value as the offset for the curve of a rate control characteristic curve (6a), which describes the dependence of the rate of the stimulation pulses on the measured variable.

7. The rate-adaptive pacemaker according to one of the preceding claims, **characterized in that** the setting device (7) has setting means (7.8 through 7.11), which are activated upon the detection of a stable state of low load, for setting the lower limiting rate (SRmin) and/or the curve of at least a section of the rate control characteristic curve (6a) according to a predetermined relationship.

8. The rate-adaptive pacemaker according to one of the preceding claims, **characterized in that** the measured variable is the respiration rate (RR) and the sensor means (1a through 4) are implemented to determine it on the basis of measurements of the intrathoracic impedance (Z).

9. The rate-adaptive pacemaker according to one of the preceding claims, **characterized in that** the measured variable is the respiration rate (RR) and the sensor means (1a through 4) are implemented to determine it on the basis of measurements of the intracardial impedance (Z).

10. The rate-adaptive pacemaker according to Claim 8 or 9, **characterized in that** the setting device is implemented in such a way that the lower limiting rate (SRmin) and/or the rate of the stimulation pulses (SRabs) is/are in an adjustable ratio to the respiration rate (RR).

11. The rate-adaptive pacemaker according to Claim 10, **characterized in that** the setting device is implemented in such a way that the lower limiting rate (SRmin) and/or the rate of the stimulation pulses (SRabs) is/are in a settable ratio to a long-term mean value of the respiration rate (RR).

12. The rate-adaptive pacemaker according to one of the preceding claims, **characterized in that** the setting device (7) is implemented for additional calibration of the rate control characteristic curve in a stable state of moderate load.

13. The rate-adaptive pacemaker according to Claims 9 and 10, **characterized in that** the sensor means are implemented for detecting the respiration volume and the state detection means (7.2 through 7.7) and/or the setting means (7.8 through 7.11) are implemented for analyzing it in addition to the respiration rate in a stable state of moderate load.

14. The rate-adaptive pacemaker according to one of the preceding claims, **characterized in that** further sensor means are provided for detecting a further measured variable as a function of activity or load and the setting device is implemented for setting the rate control characteristic curve as a function of the further measured variable.

15. The rate-adaptive pacemaker according to Claim 1, **characterized in that** the further sensor means are implemented for detecting a load-dependent measured variable in a cardiac activity signal.

## Revendications

1. Pacemaker cardiaque à fréquence adaptable (1) comportant des moyens de détection (1a à 4) pour l'enregistrement d'au moins une grandeur de mesure (RR) en relation avec la fonction circulatoire dans le corps du patient, des moyens de stimulation (8, 2) pour la génération et l'émission d'impulsions de stimulation vers le coeur (H) du patient et des moyens de contrôle de fréquence (5) qui sont reliés côté entrée aux moyens de détection et côté sortie aux moyens de stimulation, pour le contrôle de la fréquence des impulsions de stimulation (SRabs) en fonction de la grandeur de mesure en relation avec la fonction circulatoire, dans lequel les moyens de commande de fréquence présentent un dispositif de réglage (7) qui est conçu pour établir, dans un état stable de sollicitation corporelle faible ou moyenne, une grandeur de réglage pour le réglage d'une valeur absolue d'une fréquence limite inférieure (SRmin) des impulsions de stimulation en fonction de la fréquence respiratoire (RR) et pour utiliser la commande de fréquence de manière à ce que la fréquence des impulsions de stimulation (SRabs) dépende de l'évolution d'une caractéristique de contrôle de fréquence (6a) décrivant la dépendance de la fréquence des impulsions de stimulation (SRabs) par rapport à la grandeur de mesure et en plus de la grandeur de réglage déterminée pendant un état stable de sollicitation corporelle faible ou moyenne pour le réglage d'une valeur absolue d'une fréquence limite inférieure (SRmin).

2. Pacemaker cardiaque à fréquence adaptable selon la revendication 1, **caractérisé en ce que** les moyens de contrôle de fréquence (5) sont réalisés de manière à faire varier une composante dynamique de la fréquence des impulsions de stimulation en fonction d'une grandeur de mesure allant de pair avec le besoin corporel momentané par rapport à une composante statique qui dépend, par le biais du dispositif de réglage (7), de la grandeur de réglage déterminée pendant une phase de repos.

3. Pacemaker cardiaque à fréquence adaptable selon la revendication 1 ou 2, **caractérisé en ce que** le dispositif de réglage (7) comporte des moyens d'enregistrement d'état (7.2 à 7.7) pour la détection d'un état de faible sollicitation corporelle, notamment sur la base d'une comparaison d'une propriété de la grandeur de mesure avec un critère prédéfini.

4. Pacemaker cardiaque à fréquence adaptable selon la revendication 3, **caractérisé en ce que** les moyens d'enregistrement d'état (7.2 à 7.7) comprennent des moyens (7.2 à 7.5) de constatation de l'appartenance des valeurs momentanées de la grandeur de mesure à une certaine plage de valeurs.

5. Pacemaker cardiaque à fréquence adaptable selon la revendication 3 ou 4, **caractérisé en ce que** les moyens d'enregistrement d'état (7.2 à 7.7) comprennent des moyens (7.6, 7.7) de détermination de la variabilité de la grandeur de mesure et de comparaison avec une valeur limite prédéfinie.

6. Pacemaker cardiaque à fréquence adaptable selon une des revendications 1 à 5, **caractérisé en ce que** le dispositif de réglage (7) est conçu pour établir une valeur de réglage sous forme d'un décalage pour l'évolution d'une caractéristique de contrôle de fréquence (6a) décrivant la dépendance de la fréquence des impulsions de stimulation par rapport à la grandeur de mesure.

7. Pacemaker cardiaque à fréquence adaptable selon une des revendications précédentes, **caractérisé en ce que** le dispositif de réglage (7) comprend des moyens de réglage (7.8 à 7.11) pour le réglage de la fréquence limite inférieure (SRmin) et/ou de l'évolution d'au moins une section de la caractéristique de contrôle de fréquence (6a) suivant une relation prédéfinie et qui sont activés en cas de détection d'un état stable de faible sollicitation.

8. Pacemaker cardiaque à fréquence adaptable selon une des revendications précédentes, **caractérisé en ce que** la grandeur de mesure est la fréquence respiratoire (RR) et que les moyens de détection (1a à 4) servant à la déterminer sont constitués sur la base de mesures de l'impédance intrathoracique (Z).

9. Pacemaker cardiaque à fréquence adaptable selon une des revendications précédentes, **caractérisé en ce que** la grandeur de mesure est la fréquence respiratoire (RR) et que les moyens de détection (1a à 4) servant à la déterminer sont constitués sur la base de mesures de l'impédance intracardiaque (Z).

10. Pacemaker cardiaque à fréquence adaptable selon la revendication 8 ou 9, **caractérisé en ce que** le dispositif de réglage est réalisé de manière à ce que la valeur limite inférieure (SRmin) et/ou la fréquence des impulsions de stimulation (SRabs) soit en rapport réglable avec la fréquence respiratoire (RR).

11. Pacemaker cardiaque à fréquence adaptable selon la revendication 10 9, **caractérisé en ce que** le dispositif de réglage est réalisé de manière à ce que la valeur limite inférieure (SRmin) et/ou la fréquence des impulsions de stimulation (SRabs) soit en rapport réglable avec une valeur moyenne sur le long terme de la fréquence respiratoire (RR).

12. Pacemaker cardiaque à fréquence adaptable selon une des revendications précédentes, **caractérisé en ce que** le dispositif de réglage (7) est conçu pour effectuer un calibrage supplémentaire de la caractéristique de contrôle de fréquence dans un état stable de sollicitation moyenne.

13. Pacemaker cardiaque à fréquence adaptable selon les revendications 9 et 10, **caractérisé en ce que** les moyens de détection servant à enregistrer le mouvement respiratoire et les moyens d'enregistrement d'état (7.2 à 7.7) et/ou les moyens de réglage (7.8 à 7.11) servant à son exploitation, outre la fréquence respiratoire, sont constitués dans l'état stable de sollicitation moyenne.

14. Pacemaker cardiaque à fréquence adaptable selon une des revendications précédentes, **caractérisé en ce qu'**il est prévu d'autres moyens de détection pour enregistrer une autre grandeur de mesure dépendant de l'activité ou de la sollicitation et que le dispositif de réglage servant au réglage de la caractéristique de contrôle de fréquence est constitué en fonction de l'autre grandeur de mesure.

15. Pacemaker cardiaque à fréquence adaptable selon la revendication 1, **caractérisé en ce que** les autres moyens de détection sont conçus pour enregistrer une grandeur de mesure dépendant de la sollicitation dans un signal d'activité cardiaque.
